(19) Europäisches Patentamt
European Patent Office
Office européen des brevets

(11) Veröffentlichungsnummer: **0 061 037**
**A1**

(12) **EUROPÄISCHE PATENTANMELDUNG**

(21) Anmeldenummer: **82101712.6**

(22) Anmeldetag: **05.03.82**

(51) Int. Cl.³: **A 01 N 57/08**
C 07 F 9/65, C 07 D 261/06

(30) Priorität: **19.03.81 DE 3110767**

(43) Veröffentlichungstag der Anmeldung:
**29.09.82 Patentblatt 82/39**

(84) Benannte Vertragsstaaten:
**DE FR GB IT**

(71) Anmelder: **BASF Aktiengesellschaft**
**Carl-Bosch-Strasse 38**
**D-6700 Ludwigshafen(DE)**

(72) Erfinder: **Theobald, Hans, Dr. Chem.**
**Parkstrasse 2**
**D-6703 Limburgerhof(DE)**

(72) Erfinder: **Pommer, Ernst-Heinrich, Dr. Biologe**
**Berliner Platz 7**
**D-6703 Limburgerhof(DE)**

(54) **Fungizide Mittel auf Basis von 1,2-Oxazolylalkylphosphaten und die Verwendung dieser Wirkstoffe zur Bekämpfung von Pilzen.**

(57) Die vorliegende Erfindung betrifft die Verwendung von 1,2-Oxazolylalkylphosphaten der Formel

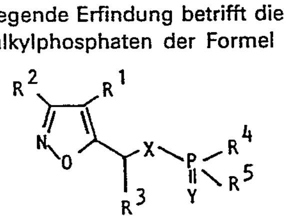

(1),

in der $R^1$, $R^2$, $R^3$, $R^4$, $R^5$, X und Y die in der Beschreibung genannten Bedeutungen haben, zur Bekämpfung von Pilzen.

**EP 0 061 037 A1**

Croydon Printing Company Ltd.

Fungizide Mittel auf Basis von 1,2-Oxazolylalkylphosphaten und die Verwendung dieser Wirkstoffe zur Bekämpfung von Pilzen

Die vorliegende Erfindung betrifft die Verwendung von 1,2-Oxazolylalkylphosphaten zur Bekämpfung von Pilzen.

1,2-Oxazolylalkylphosphate und ihre Verwendung zur Bekämpfung von Schädlingen aus der Klasse der Insekten und Spinnentiere sind aus der GB-PS 1 261 158 und der US-PS 4 212 861 bekannt.

Es wurde gefunden, daß 1,2-Oxazolylalkylphosphate der Formel

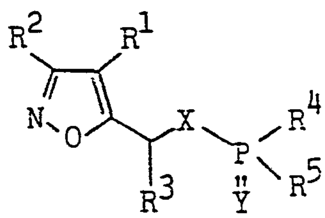

(I),

in der

$R^1$ Wasserstoff oder Halogen,

$R^2$ Wasserstoff, eine Alkyl-, eine Alkoxyalkyl- oder eine Alkylthioalkylgruppe mit bis zu 6 Kohlenstoffatomen, einen gegebenenfalls durch Alkylgruppen, Halogen oder Nitro substituierten Phenyl- oder Phenylalkylrest oder einen Alkoxycarbonylrest mit bis zu 4 Kohlenstoffatomen in der Alkoxygruppe,

$R^3$ Wasserstoff oder eine Alkylgruppe mit bis zu 3 Kohlenstoffatomen,

$R^4$ eine Alkyl- oder Alkoxygruppe mit bis zu 6 Kohlenstoffatomen oder Phenyl,

$R^5$ eine Alkoxy-, eine Alkoxyalkyl-, eine Alkylthioalkylthio-, eine Alkylthio-, Alkenylthio-, Alkinylthio-, Cycloalkoxy- oder Cycloalkylthiogruppe mit bis zu 6 Kohlenstoffatomen, Benzylthio, Amino, eine Alkylamino- oder Dialkylaminogruppe mit bis zu 4

H/P

BAD ORIGINAL

Kohlenstoffatomen in einer Alkylgruppe und X und Y unabhängig voneinander Sauerstoff oder Schwefel bedeuten, sich zur Bekämpfung von Pilzen eignen. Sie zeigen eine gute fungizide Wirksamkeit und Pflanzenverträglichkeit und eignen sich insbesondere zur Bekämpfung von Pyricularia oryzae an Reis.

Die Alkyl-, Alkoxy-, Alkylthio-, Alkenylthio-, Alkinylthio-, Alkoxyalkyl- und Alkylthioalkylgruppen für $R^2$, $R^3$, $R^4$ und $R^5$ in Formel I können unverzweigt oder verzweigt sein. Als Alkylreste kommen für $R^2$ und $R^4$ Reste mit bis zu 6 Kohlenstoffatomen, vorzugsweise mit bis zu 4 Kohlenstoffatomen, wie Methyl, Ethyl, n-Propyl, i-Propyl, n-Butyl, i-Butyl, sec.-Butyl, tert.-Butyl, n-Pentyl, i-Pentyl, i-Hexyl, als Alkylreste für $R^3$ solche mit bis zu 3 Kohlenstoffatomen, vorzugsweise Methyl, in Betracht.

Alkoxyreste für $R^4$ und $R^5$ sowie Alkylthio-, Alkenylthio-, Alkinylthio-, Cycloalkoxy- oder Cycloalkylthioreste für $R^5$ sind Reste mit bis zu 6 Kohlenstoffatomen, vorzugsweise mit bis zu 4 Kohlenstoffatomen, beispielsweise Methoxy, Ethoxy, n-Propoxy, i-Propoxy, n-Butoxy, Cyclopentoxy, Cyclohexoxy, Methylthio, Ethylthio, i-Propylthio, n-Propylthio, sec-Butylthio, i-Butylthio, tert.-Butylthio, Cyclopentylthio, Cyclohexylthio, Prop-2-enylthio, But-2-enylthio, But-3-enylthio, Propargylthio, Butin-2-ylthio.

Geeignete Alkoxyalkylreste mit bis zu 6 Kohlenstoffatomen für $R^2$ und $R^5$ sind Methoxymethyl, Methoxyethyl, Ethoxymethyl, Ethoxyethyl, geeignete Alkylthioalkylthioreste für $R^5$ sind Methylthiomethylthio, Methylthioethylthio, Ethylthiomethylthio, Ethylthioethylthio.

$R^2$ kann außerdem für einen gegebenenfalls durch Alkylgruppen mit bis zu 4 Kohlenstoffatomen, wie Methyl, Ethyl, i-Propyl,

Halogen, wie Fluor, Chlor, Brom, insbesondere Chlor, oder Nitro einfach oder mehrfach substituierten Phenyl- oder Phenylalkylrest stehen, beispielsweise für Benzyl, Phenethyl, 2-Phenyl-i-propyl, 3,4-Dichlorphenyl, 2-(4-i-Propyl-phenyl)-i-propyl, 2-(4-tert.-Butyl-phenyl)-i-propyl, 3-Nitrophenyl, oder für einen Alkoxycarbonylrest mit bis zu 4 Kohlenstoffatomen in der Alkoxygruppe, wie Methoxycarbonyl, Ethoxycarbonyl, i-Propoxycarbonyl, stehen. Alkylamino- oder Dialkylaminogruppen mit bis zu 4 Kohlenstoffatomen in einer Alkylgruppe für $R^5$ sind beispielsweise Methylamino, Dimethylamino, i-Propylamino.

Als Halogen für $R^1$ kommen Fluor, Chlor, Brom oder Jod, insbesondere Chlor oder Brom, in Betracht.

Man erhält die 1,2-Oxazolylalkylphosphate der Formel I, indem man Salze von Phosphorsäurederivaten der Formel II mit 1,2-Oxazolderivaten der Formel III nach folgendem Reaktionsschema umsetzt:

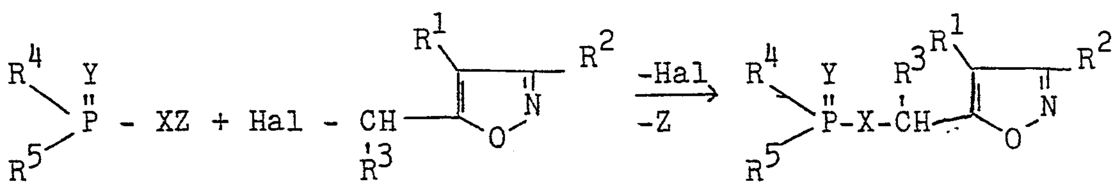


II III I

Dabei haben die Reste $R^1$, $R^2$, $R^3$, $R^4$, $R^5$, X und Y die oben angegebenen Bedeutungen, Hal steht für ein Halogenatom und Z für ein Alkaliion, ein Äquivalent Erdalkaliion oder ein gegebenenfalls durch Alkylreste substituiertes Ammoniumion (US-PS 4 212 861).

Als Halogen kommen Fluor, Chlor, Brom, Jod in Betracht, bevorzugt sind Chlor und Brom. Als Alkaliionen sind Natrium und Kalium, als Erdalkaliionen Magnesium und Calcium und als Ammoniumionen das unsubstituierte Ion, Methyl-, Ethyl-, Propyl-, Isopropyl-, Dimethyl-, Diethyl-, Trimethyl-, Triethyl-, Tetramethyl- oder Tetraethylammonium bevorzugt.

Die als Ausgangsverbindungen verwendeten 1,2-Oxazolderivate der Formel III können durch 1,3-dipolare Cycloaddition von Nitriloxiden an Acetylenverbindungen erhalten werden (DE-OS 25 49 962). Die phosphorsauren Salze der Formel II können nach an sich bekannten Verfahren hergestellt werden (Houben-Weyl, Methoden der organischen Chemie, Band 12/2, S. 131 ff. Georg Thieme-Verlag, Stuttgart, 1964).

Die Umsetzung wird im allgemeinen in Anwesenheit von Verdünnungsmitteln ausgeführt. Solche Verdünnungsmittel sind beispielsweise Wasser, niedere Alkohole, wie Methanol, Ethanol, Propanol, Nitrile, wie Acetonitril, Ketone, wie Aceton, Methylethylketon, Ether, wie Dioxan, Tetrahydrofuran, aromatische Verbindungen, wie Benzol, Toluol, Xylole und Chlorbenzole, Dimethylformamid, Dimethylsulfoxid. Zur Umsetzung kann man beide Reaktionspartner in äquimolarem Verhältnis oder einen der beiden Reaktionspartner im Überschuß einsetzen. Die Reaktionstemperaturen liegen bei 0 bis 150°C, vorzugsweise im Temperaturbereich von 20 bis 100°C.

Zur Charakterisierung der Verbindungen dienen NMR-spektroskopische Daten ($\delta$-Werte; $CDCl_3$ als Lösungsmittel).

Folgende 1,2-Oxazolylalkylphosphate der Formel I können beispielsweise zur Bekämpfung von Pilzen verwendet werden:

| Nr. | $R^1$ | $R^2$ | $R^3$ | $R^4$ | $R^5$ | X | Y | NMR-Daten (MHz) δ-Werte |
|---|---|---|---|---|---|---|---|---|
| 1 | H | $CH_3$ | H | $C_2H_5O$ | $n-C_3H_7S$ | S | O | (60 MHz) 0,85 (3 H); 1,18 (3 H); 1,54 (2 H); 2,15 (3 H); 2,66 (2 H); 3,8-4,2 (4 H); 6,03 (1 H) |
| 2 | H | $CH_3$ | H | $C_2H_5O$ | $C_2H_5O$ | O | S | (60 MHz) 1,3 (6 H); 2,22 (3 H); 3,8-4,4 (4 H) 5,1 (2 H); 6,2 (1 H) |
| 3 | H | $CH_3$ | H | $CH_3O$ | $CH_3O$ | O | S | (100 MHz) 2,3 (3 H); 3,74 (6 H); 5,14 (2 H); 6,2 (1 H) |
| 4 | H | $CH_3$ | H | $C_2H_5O$ | $C_2H_5O$ | O | O | (220 MHz) 1,28 (6 H); 2,1 (3 H); 3,6-3,9 (4 H); 4,6 (2 H); 5,65 (1 H) |
| 5 | H | $CH_3$ | H | $CH_3O$ | $CH_3O$ | S | O | (220 MHz) 2,15 (3 H); 3,48 (6 H); 3,74 (2 H); 5,6 (1 H) |
| 6 | H | $CH_3$ | H | $C_2H_5O$ | $CH_3S$ | S | O | (60 MH7) 1,32 (3 H); 2,24 (3 H); 2,46 (3 H); 3,9-4,4 (4 H); 6,12 (1 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 7 | H | $CH_3$ | H | $C_2H_5O$ | $n-C_4H_9S$ | S | O | (60 MHz) 0,94 (3 H); 1,35 (3 H); 1,2-1,8 (4 H); 2,23 (3 H); 2,83 (2 H); 3,9-4,35 (4 H); 4,12 (1H) |
| 8 | H | $CH_3$ | H | $n-C_3H_7O$ | $n-C_3H_7O$ | S | S | (60 MHz) 0,9 (6 H); 1,68 (4 H); 2,21 (3 H); 3,7-4,28 (6 H); 6,06 (1 H) |
| 9 | H | $CH_3$ | H | $n-C_4H_9O$ | $n-C_4H_9O$ | S | S | (60 MHz) 0,8-1,2 (6 H); 1,25-1,95 (8 H); 2,3 (3 H); 3,9-4,37 (6 H); 6,14 (1 H) |
| 10 | H | $CH_3$ | H | $n-C_3H_7O$ | $n-C_3H_7S$ | S | O | (60 MHz) 0,8-1,17 (6 H); 1,42-2,0 (4 H); 2,3 (3 H); 2,75-3,2 (2 H); 3,85-4,33 (4 H); 6,14 (1 H) |
| 11 | H | $CH_3$ | H | $CH_3O$ | Benzylthio | S | O | (80 MHz) 2,2 (3 H); 3,65 (3 H); 3,9-4,2 (4 H) 6,02 (1 H); 7,2 (5 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 12 | H | $CH_3$ | H | $CH_3O$ | $CH_3O$ | O | O | (60 MHz) 2,25 (3 H); 3,75 (6 H); 6,24 (1 H) |
| 13 | H | $CH_3$ | H | i-$C_3H_7O$ | i-$C_3H_7O$ | O | O | (60 MHz) 1,2-1,5 (12 H); 2,3 (3 H); 4,65 (2 H); 5,1 (2 H); 6,24 (1H) |
| 14 | H | $CH_3$ | H | $C_2H_5O$ | i-$C_3H_7O$ | S | O | (220 MHz) 1,3 (3 H); 1,34 (6 H); 2,11 (3 H); 3,24 (1 H); 3,82-4,91 (2 H + 2 H) |
| 15 | H | H | H | $C_2H_5O$ | $C_2H_5O$ | S | S | (100 MHz) 1,2-1,5 (6 H); 3,9-4,4 (4 H + 2 H); 6,5 (1 H); 8,46 (1 H) |
| 16 | H | H | H | $C_2H_5O$ | n-$C_3H_7S$ | S | O | (100 MHz) 1,05 (3 H); 1,38 (3 H); 1,75 (2 H); 3,95 (2 H); 4,1-4,4 (2 H + 2 H); 6,55 (1 H); 8,48 (1 H) |
| 17 | H | $C_2H_5$ | H | $C_2H_5O$ | $C_2H_5O$ | S | S | (60 MHz) 1,1-1,5 (9 H); 2,7 (2 H); 4,13 (2 H); 3,8-4,4 (4 H); 6,17 (1 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 18 | H | $C_2H_5$ | H | $C_2H_5O$ | n-$C_3H_7S$ | S | O | (60 MHz) 0,98 (3 H); 1,23 (3 H); 1,33 (3 H); 1,68 (2 H); 2,4-3,15 (4 H); 3,85-4,32 (4 H); 6,17 (1 H) |
| 19 | H | 3,4-Dichlor-phenyl | H | $C_2H_5O$ | $C_2H_5O$ | S | S | (60 MHz) 1,3 (6 H); 3,9-4,45 (4 H + 2 H); 6,25 (1 H); 7,4-7,6 (2 H); 7,75-7,9 (1 H) |
| 20 | H | 3,4-Dichlor-phenyl | H | $C_2H_5O$ | n-$C_3H_7S$ | S | O | (60 MHz) 0,95 (3 H); 1,35 (3 H); 1,7 (2 H); 2,9 (2 H); 4,0-4,6 (2 H + 2 H); 6,6 (1 H); 7,4-7,6 (2 H); 7,8-7,9 (1 H) |
| 21 | H | 3,4-Dichlor-phenyl | H | $C_2H_5O$ | i-$C_3H_7S$ | S | O | (60 MHz) 1,1-1,5 (6 H); 3,3-3,6 (1 H); 3,9-4,5 (2 H + 2 H); 7,1 (1 H); 7,6-8,1 (3 H) |
| 22 | H | $C_2H_5$ | H | $C_2H_5O$ | $C_2H_5S$ | O | S | (60 MHz) 1,1 (3 H); 1,2 (6 H); 2,55 (2 H); 3,9-4,2 (4 H); 5,06 (2 H); 6,37 (1 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 23 | H | 3,4-Dichlor-phenyl | H | $CH_3O$ | $CH_3O$ | S | S | (60 MHz) 3,6 (6 H); 4,2 (2 H); 6,9 (1 H); 7,4-7,9 (3 H) |
| 24 | H | $C_2H_5$ | H | $CH_3O$ | $CH_3O$ | S | O | (60 MHz) 1,22 (3 H); 2,63 (2 H); 3,71 (6 H); 4,06 (2 H); 6,14 (H) |
| 25 | H | $CH_3$ | H | $C_2H_5O$ | s-$C_4H_9S$ | S | O | (60 MHz) 0,96 (3 H); 1,28 (3 H); 1,4 (3 H); 1,64 (2 H); 2,25 (3 H); 3,3 (H); 4,12 (2 H); 4,2 (2 H); 6,16 (H) |
| 26 | H | $C_2H_5$ | H | $C_2H_5O$ | s-$C_4H_9S$ | S | O | (60 MHz) 0,98 (3 H); 1,21 (3 H); 1,36 (3 H + 3 H); 1,66 (2 H); 2,68 (2 H); 3,37 (1 H); 4,19 (2 H); 4,32 (2 H); 6,19 (1 H) |
| 27 | H | $CH_3$ | H | $C_2H_5O$ | $CH_3SCH_2CH_2S$ | S | O | (80 MHz) 1,35 (3 H); 2,0-2,2 (3 H + 3 H); 2,2-3,3 (2 H + 2 H); 4,0-4,3 (2 H + 2 H); 6,08 (1 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 28 | H | $C_2H_5$ | H | $C_2H_5O$ | $CH_3SCH_2CH_2S$ | S | O | (60 MHz) 1,2 (3 H); 1,4 (3 H); 2,1 (3 H); 2,4-2,9 (2 H + 2 H); 3,1-3,3 (2 H); 3,9-4,3 (2 H + 2 H); 6,15 (1 H) |
| 29 | H | $CH_3$ | H | n-$C_3H_7O$ | $C_2H_5S$ | S | O | (60 MHz) 0,85 (3 H); 1,26 (3 H); 2,51 (2 H); 2,12 (3 H); 2,8 (2 H); 3,9 (2 H); 3,95 (2 H); 6,01 (H) |
| 30 | H | $C_2H_5$ | H | n-$C_3H_7O$ | $C_2H_5S$ | S | O | (60 MHz) 0,93 (3 H); 1,25 (3 H); 1,39 (3 H); 1,66 (2 H); 2,4-3,15 (4 H); 3,8-4,3 (2 H); 4,08 (2 H); 6,12 (H) |
| 31 | H | H | H | n-$C_3H_7O$ | $C_2H_5S$ | S | O | (60 MHz) 1,1 (3 H); 1,45 (3 H); 1,75 (2 H); 2,95 (2 H); 4,0-4,3 (2 H + 2 H); 6,52 (1 H); 8,4 (1 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 32 | H | i-$C_3H_7$ | H | $C_2H_5O$ | $C_2H_5O$ | S | S | (60 MHz) 1,13-1,5 (6 H + 6 H); 2,89 (H); 3,7-4,35 (4 H); 4,03 (2 H); 6,07 (H) |
| 33 | H | i-$C_3H_7$ | H | n-$C_3H_7O$ | $C_2H_5S$ | S | O | (60 MHz) 0,88 (3 H); 1,2 (6 H); 1,25 (3 H); 1,53 (2 H); 2,4-3,2 (2 H + H); 3,75-4,1 (2 H); 4,0 (2 H); (6,02 (H) |
| 34 | H | i-$C_3H_7$ | H | $C_2H_5O$ | s-$C_4H_9S$ | S | O | (60 MHz) 1,0 (3 H); 1,2-2,0 (6 H + 3H + 3H + 2 H); 2,8-3,5 (1 H + 1 H); 4,0-4,3 (2 H + 2 H), 6,17 (1 H) |
| 35 | H | t-$C_4H_9$ | H | $C_2H_5O$ | $C_2H_5O$ | S | S | (60 MHz) 1,3-1,4 (9 H + 6 H); 4,0-4,3 (2 H + 4 H); 6,2 (1 H) |
| 36 | H | t-$C_4H_9$ | H | $C_2H_5O$ | n-$C_3H_7S$ | S | O | (60 MHz) 1,05 (3 H); 1,36 (9 H + 3 H); 1,8 (2 H); 2,96 (2 H); 4,21 (2 H); 4,29 (2 H); 6,3 (1 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 37 | H | t-$C_4H_9$ | H | n-$C_3H_7O$ | $C_2H_5S$ | S | O | (60 MHz) 0,97 (3 H); 1,35 (9 H); 1,4 (3 H); 1,7 (2 H); 3,0 (2 H); 4,05-4,33 (2 H + 2 H); 6,27 (1 H) |
| 38 | H | t-$C_4H_9$ | H | $C_2H_5O$ | i-$C_3H_7S$ | S | O | (60 MHz) 1,36 (9 H); 1,47 (6 H); 3,6 (1 H); 4,05-4,3 (2 H + 2 H); 6,2 (1 H) |
| 39 | H | t-$C_4H_9$ | H | $CH_3O$ | $CH_3O$ | S | S | (100 MHz) 1,2 (9 H); 3,7 (6 H); 4,1 (2 H); 6,15 (1 H) |
| 40 | H | Phenyl | H | $C_2H_5O$ | $C_2H_5O$ | S | S | (60 MHz) 1,2 (6 H); 2,8 (2 H); 3,85-4,3 (2 H + 2 H); 6,46 (1 H); 7,2-7,8 (5 H) |
| 41 | H | Phenyl | H | $CH_3O$ | $CH_3O$ | S | S | (60 MHz) 3,6 (6 H); 4,0 (2 H); 6,4 (1 H); 7,1-7,4 (5 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 42 | H | Phenyl | H | $C_2H_5O$ | $C_3H_7S$ | S | O | (60 MHz) 1,0 (3 H); 1,3 (3 H); 1,64 (2 H); 2,86 (2 H); 4,05-4,35 (2 H + 2 H); 6,7 (1 H); 7,25-7,5 (3 H); 7,7-7,9 (2 H) |
| 43 | H | Phenyl | H | $n-C_3H_7$ | $C_2H_5S$ | S | O | (80 MHz) 0,95 (3 H); 1,3 (3 H); 1,7 (2 H); 1,86 (2 H); 4,0-4,3 (2 H + 2 H); 6,5 (1 H); 7,2-7,4 (3 H); 7,5-7,7 (2 H) |
| 44 | H | $n-C_3H_7$ | H | $C_2H_5O$ | $C_2H_5O$ | S | S | (60 MHz) 1,0 (3 H); 1,34 (6 H); 1,7 (2 H); 2,66 (2 H); 4,0-4,3 (2 H + 4 H); 6,13 (1 H) |
| 45 | H | $n-C_3H_7$ | H | $C_2H_5O$ | $n-C_3H_7S$ | S | O | (60 MHz) 1,04 (6 H); 1,42 (3 H); 1,5-2,1 (4 H); 2,7 (2 H); 3,01 (2 H); 4,05-4,3 (2 H + 2 H); 6,19 (1 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 46 | H | n-$C_3H_7$ | H | n-$C_3H_7O$ | $C_2H_5S$ | S | O | (60 MHz) 1,0 (6 H); 1,52 (3 H); 1,5-2,0 (4 H); 2,68 (2 H); 3,0 (2 H); 4,03-4,3 (2 H + 2 H); 6,18 (1 H) |
| 47 | H | s-$C_4H_9$ | H | $C_2H_5O$ | $C_2H_5O$ | S | S | (60 MHz) 0,86 (3 H); 1,18 (3 H); 1,3 (6 H); 1,55 (2 H); 2,83 (1 H); 3,93-4,3 (4 H + 2 H); 6,1 (1 H) |
| 48 | H | s-$C_4H_9$ | H | $CH_3O$ | $CH_3O$ | S | S | (60 MHz) 0,9 (3 H); 1,22 (3 H); 1,55 (2 H); 2,8 (1 H); 3,74 (6 H); 4,1 (2 H); 6,1 (1 H) |
| 49 | H | s-$C_4H_9$ | H | $C_2H_5O$ | n-$C_3H_7S$ | S | O | (60 MHz) 0,8 (3 H); 0,9 (3 H); 1,05 (3 H); 1,2 (3 H); 1,8-1,9 (2 H + 2 H); 2,5-3,05 (2 H + 1 H); 3,9-4,3 (2 H + 1 H); 6,05 (1 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 50 | H | s-$C_4H_9$ | H | n-$C_3H_7O$ | $C_2H_5S$ | S | O | (60 MHz) 0,8 (3 H); 0,88 (3 H); 1,1 (3 H); 1,24 (3 H); 1,3 (2 H); 1,65 (2 H); 2,5-3,0 (2 H + 1 H); 3,8-4,2 (2 H + 2 H); 6,05 (1 H) |
| 51 | H | s-$C_4H_9$ | H | $CH_3O$ | $CH_3O$ | S | O | (60 MHz) 0,88 (3 H); 1,27 (3 H); 1,6 (2 H); 2,8 (1 H); 3,78 (6 H); 4,07 (2 H); 6,1 (1 H) |
| 52 | H | $CH_3OCH_2$ | H | $C_2H_5O$ | $C_2H_5O$ | S | S | (60 MHz) 1,33 (6 H); 3,37 (3 H); 3,94-4,3 (4 H + 2 H); 4,05 (2 H); 6,3 (1 H) |
| 53 | H | $CH_3OCH_2$ | H | $CH_3O$ | $CH_3O$ | S | S | (60 MHz) 3,08 (3 H); 3,7 (6 H); 4,05 (2 H); 4,4 (2 H); 6,2 (1 H) |
| 54 | H | $CH_3OCH_2$ | H | n-$C_3H_7O$ | $C_2H_5O$ | S | O | (60 MHz) 0,91 (3 H); 1,30 (3 H); 1,6 (2 H); 2,83 (2 H); 3,3 (3 H); 3,9-4,3 (2 H + 2 H); 4,21 (2 H); 6,25 (1 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 55 | H | $CH_3OCH_2$ | H | $C_2H_5O$ | n-$C_3H_7S$ | S | O | (60 MHz) 1,1 (3 H); 1,35 (3 H); 1,75 (2 H); 2,9 (2 H); 3,38 (3 H); 4,05-4,35 (4 H); 4,5 (2 H); 6,34 (1 H) |
| 56 | H | $CH_3OCH_2$ | H | $C_2H_5O$ | i-$C_3H_7S$ | S | O | (60 MHz) 1,34 (3 H); 1,4 (6 H); 3,3 (6 H); 3,5 (1 H); 3,9-4,2 (2 H + 2 H); 4,3 (2 H); 6,27 (1 H) |
| 57 | H | $CH_3OCH_2$ | H | $CH_3O$ | $CH_3O$ | S | O | (60 MHz) 3,3 (3 H); 3,7 (6 H); 4,3 (2 H); 4,4 (2 H); 6,22 (1 H) |
| 58 | H | i-$C_4H_9$ | H | $C_2H_5O$ | $C_2H_5O$ | O | O | (60 MHz) 0,98 (6 H); 1,3 (6 H); 1,9 (1 H); 2,45 (2 H); 3,9-4,3 (4 H + 2 H); 6,02 (1 H) |
| 59 | H | Phenyl | H | $C_2H_5O$ | i-$C_3H_7S$ | S | S | (60 MHz) 1,16 (3 H); 1,35 (6 H); 2,82 (1 H); 3,5 (1 H); 3,9-4,3 (2 H + 2 H); 6,5 (1 H); 7,2-7,4 (3 H); 7,58-7,8 (2 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 60 | H | s-$C_4H_9$ | H | $C_2H_5O$ | i-$C_3H_7S$ | S | O | (60 MHz) 0,88 (3 H); 1,24 (3 H); 1,3 (3 H); 1,4 (6 H); 1,6 (2 H); 2,84 (1 H); 3,54 (1 H); 4,0-4,5 (2 H + 2 H); 6,14 (1 H) |
| 61 | H | i-$C_4H_9$ | H | $CH_3O$ | $CH_3O$ | S | S | (60 MHz) 0,83 (6 H); 1,8 (1 H); 2,86 (2 H); 3,6 (6 H); 3,9 (2 H); 5,96 (1 H) |
| 62 | H | i-$C_4H_9$ | H | $C_2H_5O$ | n-$C_3H_7S$ | S | O | (60 MHz) 0,84 (6 H); 0,9 (3 H); 1,23 (3 H); 1,4-2,05 (2 H + 1 H); 2,4 (2 H); 2,77 (2 H); 3,88-4,22 (2 H + 2 H); 6,04 (1 H) |
| 63 | H | i-$C_4H_9$ | H | n-$C_3H_7O$ | $C_2H_5S$ | S | O | (60 MHz) 0,8-1,0 (3 H + 6 H); 1,25 (3 H); 1,58 (2 H); 1,7 (1 H); 3,82-4,2 (2 H + 2 H); 5,98 (1 H) |

| Nr. | $R^1$ | $R^2$ | R | $R^4$ | $R^5$ | X | Y | NMR-Daten (MHz) δ-Werte |
|---|---|---|---|---|---|---|---|---|
| 64 | H | n-$C_3H_7$ | H | $CH_3O$ | $CH_3O$ | S | O | (60 MHz) 0,86 (3 H); 1,52 (2 H); 2,54 (2 H); 3,66 (6 H); 4,25 (2 H); 6,0 (1 H) |
| 65 | H | n-$C_3H_7$ | H | $C_2H_5O$ | 1-$C_3H_7S$ | S | O | (60 MHz) 0,97 (3 H); 1,3 (3 H); 1,6 (6 H); 2,62 (2 H); 3,57 (1 H); 4,0-4,3 (2 H + 2 H); 6,14 (1 H) |
| 66 | H | n-$C_3H_7$ | H | $CH_3O$ | $CH_3O$ | S | S | (60 MHz) 0,94 (3 H); 1,66 (2 H); 2,56 (2 H); 3,72 (6 H); 4,1 (2 H); 6,1 (1 H) |
| 67 | H | 1-$C_4H_9$ | H | $C_2H_5O$ | 1-$C_3H_7S$ | S | O | (60 MHz) 0,9 (6 H); 1,22 (3 H); 1,3 (6 H); (1,84 (1 H); 2,38 (2 H); 3,39 (1 H); 3,9-4,3 (2 H + 2 H); 6,0 (1 H) |
| 68 | H | n-$C_4H_9$ | H | $C_2H_5O$ | $C_2H_5O$ | S | S | (60 MHz) 0,9 (3 H); 1,24 (6 H); 1,2-1,8 (4 H); 2,5 (2 H); 3,8-4,2 (2 H + 4 H); 6,02 (1 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 69 | H | n-$C_4H_9$ | H | $CH_3O$ | $CH_3O$ | S | S | (60 MHz) 0,95 (3 H); 1,1-1,7 (4 H); 2,55 (2 H); 3,65 (6 H); 4,0 (2 H); 6,05 (1 H) |
| 70 | H | n-$C_4H_9$ | H | $C_2H_5O$ | n-$C_3H_7S$ | S | O | (60 MHz) 0,66-1,0 (6 H); 1,1-1,85 (3 H + 4 H + 2 H); 2,46 (2 H); 2,82 (2 H); 3,9 (2 H); 4,01 (2 H); 6,06 (1 H) |
| 71 | H | 1-$C_3H_7$ | H | $CH_3O$ | $CH_3O$ | S | S | (60 MHz) 1,1 (6 H); 2,88 (1 H); 3,73 (6 H); 3,95 (2 H); 6,02 (1 H) |
| 72 | H | n-$C_4H_9$ | H | n-$C_3H_7O$ | $C_2H_5S$ | S | O | (60 MHz) 0,76 (3 H); 0,82 (3 H); 1,2 (3 H); 1,05-1,82 (4 H + 2 H); 2,25 (2 H); 2,87 (2 H); 3,8-4,2 (2 H + 2 H); 6,02 (1 H) |
| 73 | H | n-$C_4H_9$ | H | $CH_3O$ | $CH_3O$ | S | O | (60 MHz) 0,9 (3 H); 1,1-1,7 (4 H); 2,5 (2 H); 3,7 (6 H); 4,25 (2 H); 6,0 (1 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 74 | H | n-$C_4H_9$ | H | $C_2H_5O$ | i-$C_3H_7S$ | S | O | (60 MHz) 0,9 (3 H); 1,05-1,7 (3 H + 6 H + 4 H); 2,5 (2 H); 3,42 (1 H); 4,05 (2 H); 4,12 (2 H); 6,03 (1 H) |
| 75 | H | $CH_3$ | H | $C_2H_5O$ | i-$C_4H_9S$ | S | O | (60 MHz) 0,97 (6 H); 1,32 (3 H); 1,94 (1 H); 2,5 (3 H); 2,7 (2 H); 3,9-4,2 (2 H + 2 H); 6,04 (1 H) |
| 76 | H | $CH_3OCH_2$ | H | $C_2H_5O$ | i-$C_4H_9S$ | S | O | (80 MHz) 1,04 (6 H); 1,38 (3 H); 2,84 (2 H); 3,4 (3 H); 4,05-4,3 (2 H + 2 H) 4,5 (2 H); 6,35 (1 H) |
| 77 | H | t-$C_4H_9$ | H | $C_2H_5O$ | i-$C_4H_9S$ | S | O | (60 MHz) 0,98 (6 H); 1,12-1,4 (9 H + 3 H); 1,9 (1 H); 2,82 (2 H); 3,9-4,2 (2 H + 2 H); 6,12 (1 H) |
| 78 | H | 3-Nitrophenyl | H | $C_2H_5O$ | $C_2H_5O$ | S | S | (80 MHz) 1,3 (6 H); 3,85-4,4 (4 H); 6,6 (1 H); 7,4-8,3 (4 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 79 | H | 3-Nitrophenyl | H | $C_2H_5O$ | n-$C_3H_7S$ | S | O | (60 MHz) 0,98 (3 H); 1,4 (3 H); 1,74 (2 H); 2,9 (2 H); 4,1-4,4 (4 H); 6,8 (1 H); 7,5-8,7 (4 H) |
| 80 | H | 3-Nitrophenyl | H | $C_2H_5O$ | sec-$C_4H_9S$ | S | O | (80 MHz) 1,0 (3 H); 1,2-1,6 (3 H + 2 H); 1,75 (2 H); 3,42 (1 H); 4,2 (2 H); 4,4 (2 H); 6,8 (1 H); 7,5-8,6 (4 H) |
| 81 | H | 2-(4-t-Butyl-phenyl)-1-propyl | H | $C_2H_5O$ | n-$C_3H_7S$ | S | O | (80 MHz) 1,0 (3 H); 1,25 (9 H); 1,7 (2 H); 2,6-3,1 (3 H + 2 H); 3,9-4,25 (2 H + 1 H); 6,0 (1 H); 6,9-7,2 (4 H) |
| 82 | H | $C_2H_5$ | H | $C_2H_5O$ | i-$C_4H_9S$ | S | O | (100 MHz) 1,0 (6 H); 1,21 (3 H); 1,35 (3 H); 1,92 (1 H); 2,68 (2 H); 2,82 (2 H); 4,0-4,3 (2 H + 2 H); 1,13 (1 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 83 | H | 1-$C_3H_7$ | H | $C_2H_5O$ | 1-$C_4H_9S$ | S | O | (100 MHz) 1,0 (6 H); 1,3 (6 H); 1,34 (3 H); 1,93 (1 H); 2,78 (2 H); 2,94 (1 H); 4,0-4,3 (2 H + 2 H); 6,14 (1 H) |
| 84 | H | Phenyl | H | $C_2H_5O$ | 1-$C_4H_9S$ | S | O | (60 MHz) 1,0 (6 H); 1,3 (3 H); 1,9 (1 H); 2,8 (2 H); 4,0-4,3 (2 H + 2 H); 6,52 (1 H); 7,2-7,5 (3 H); 7,6-7,8 (2 H) |
| 85 | H | 1-$C_3H_7$ | H | $C_2H_5O$ | 1-$C_4H_9S$ | S | O | (60 MHz) 0,8-1,2 (12 H); 1,38 (3 H); 1,95 (2 H); 2,6 (2 H); 2,88 (2 H); 4,0-4,4 (2 H + 2 H); 6,1 (1 H) |
| 86 | H | 2-(4-i-Propyl-phenyl)-1-propyl | H | $C_2H_5O$ | $C_2H_5O$ | S | S | (270 MHz) 1,1-1,4 (3 H + 6 H + 6 H); 2,65 (1 H); 2,8 (1 H); 2,95 (1 H), 3,2 (1 H); 4,1 (2 H); 6,1 (1 H); 7,0-7,15 (4 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 87 | H | 2-(4-i-Propyl-phenyl)-1-propyl | H | $C_2H_5O$ | n-$C_3H_7S$ | S | O | (60 MHz) 0,8-1,9 (6 H + 3 H + 6 H); 2,55-3,2 (4 H + 1 H); 3,8-4,5 (2 H + 2 H); 6,08 (1 H); 6,9-7,2 (4 H) |
| 88 | H | $CH_3$ | H | $C_2H_5O$ | n-$C_4H_9O$ | S | O | (60 MHz) 0,9 (3 H); 1,3 (3 H); 1,2-1,7 (4 H); 2,26 (3 H); 3,9-4,3 (4 H + 2 H); 6,08 (1 H) |
| 89 | H | $CH_3$ | H | $C_2H_5O$ | i-$C_4H_9O$ | S | O | (220 MHz) 0,88 (6 H); 1,3 (3 H); 1,85 (1 H); 2,2 (3 H); 3,72 (2 H); 3,98-4,1 (2 H + 2 H); 6,05 (1 H) |
| 90 | H | $CH_3$ | H | $C_2H_5O$ | s-$C_4H_9O$ | S | O | (80 MHz) 0,95 (3 H); 1,2-1,45 (3 H + 3 H); 1,6 (2 H); 2,22 (3 H); 3,9-4,2 (2 H + 2 H); 4,4-4,6 (1 H); 6,1 (1 H) |
| 91 | H | $CH_3$ | H | $C_2H_5O$ | t-$C_4H_9S$ | S | O | (80 MHz) 1,31 (3 H); 1,58 (9 H); 2,26 (3 H); 3,9-4,3 (2 H + 2 H); 6,1 (1 H) |

| Nr. | $R^1$ | $R^2$ | $R^3$ | $R^4$ | $R^5$ | X | Y | NMR-Daten (MHz) σ-Werte |
|---|---|---|---|---|---|---|---|---|
| 92 | H | $CH_3$ | H | $C_2H_5O$ | $C_2H_5O$ | S | O | (60 MHz) 1,34 (6 H); 2,3 (3 H); 3,9-4,3 (4 H + 2 H); 6,1 (1 H) |
| 93 | H | $CH_3$ | H | $C_2H_5O$ | $C_5H_9S$ | S | O | (220 MHz) 1,2 (3 H); 1,4-1,7 (6 H); 2,0-2,1 (2 H); 2,3 (3 H); 3,7 (1 H); 4,1-4,25 (2 H + 2 H); 6,22 (1 H) |
| 94 | H | $t-C_4H_9$ | H | $C_2H_5O$ | $C_5H_9S$ | S | O | (100 MHz) 1,2-1,4 (9 H); 1,5-1,8 (6 H); 2,1 (2 H); 3,5 (1 H); 4,0 (2 H); 4,15 (2 H); 6,1 (1 H) |
| 95 | H | $C_2H_5OCO$ | H | $C_2H_5O$ | $C_2H_5O$ | S | S | (220 MHz) 1,2-1,4 (3 H + 3 H); 4,0-4,3 (4 H + 2 H); 4,4 (2 H); 6,6 (1 H) |
| 96 | H | $C_2H_5OCO$ | H | $C_2H_5O$ | $n-C_3H_7S$ | S | O | (100 MHz) 1,0 (3 H); 1,25-1,5 (3 H + 3 H); 1,7 (2 H); 2,85 (2 H); 4,1-4,5 (2 H + 2 H + 2 H); 6,66 (1 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 97 | H | $C_2H_5OCO$ | H | $C_2H_5O$ | $i-C_4H_9S$ | S | O | (220 MHz) 0,98 (6 H); 1,3 (3 H + 3 H); 1,9 (1 H); 2,8 (2 H); 4,0-4,2 (2 H + 2 H); 4,4 (2 H); 6,8 (1 H) |
| 98 | H | $C_2H_5OCO$ | H | $C_2H_5O$ | $s-C_4H_9S$ | S | O | (220 MHz) 0,9 (3 H); 1,2-1,4 (3 H + 3 H); 1,7 (2 H); 3,4 (1 H); 4,0-4,0 (2 H + 2 H + 2 H); 6,8 (1 H) |
| 99 | H | $CH_3$ | H | $C_2H_3C$ | $C_6H_{11}O$ | S | O | (100 MHz) 1,2-2,1 (13 H); 2,5 (3 H); 3,72 (3 H); 4,1 (2 H); 4,5 (1 H); 6,1 (1 H) |
| 100 | H | $CH_3$ | H | $CH_3O$ | $C_5H_9O$ | S | O | (100 MHz) 1,8-2,2 (8 H); 2,3 (3 H); 3,6 (1 H); 3,8 (3 H); 4,15 (2 H); 6,12 (1 H) |
| 101 | Cl | $CH_3$ | H | $C_2H_5O$ | $i-C_3H_7S$ | S | O | (60 MHz) 1,2-1,6 (9 H); 2,3 (3 H); 3,7 (1 H); 4,0-4,4 (2 H + 2 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 102 | Cl | $CH_3$ | H | $C_2H_5O$ | $CH_3-C{\equiv}C-CH_2S$ | S | O | (60 MHz) 1,3 (3 H); 1,7 (3 H); 2,14 (3 H); 3,5 (2 H); 4,1 (2 H); 4,17 (2 H) |
| 103 | Cl | $CH_3$ | H | $C_2H_5S$ | $n-C_3H_7O$ | S | O | (60 MHz) 0,96 (3 H); 1,42 (3 H); 1,75 (2 H); 2,3 (3 H); 2,95 (2 H); 4,05 (2 H); 4,15 (2 H) |
| 104 | Cl | $CH_3$ | H | $C_2H_5O$ | $n-C_3H_7O$ | S | O | (60 MHz) 0,95 (3 H); 1,28 (3 H); 1,66 (2 H); 2,13 (3 H); 2,76 (2 H); 4,1 (2 H); 4,15 (2 H) |
| 105 | Cl | $CH_3$ | H | $C_2H_5O$ | $C_2H_5O$ | S | S | (60 MHz) 1,36 (6 H); 2,25 (3 H); 3,9-4,3 (2 H + 4 H) |
| 106 | H | $CH_3$ | $CH_3$ | $C_2H_5O$ | $C_2H_5O$ | S | S | (60 MHz) 1,75 (3 H); 2,3 (3 H); 3,8-4,8 (2 H + 2 H + 1 H); 6,05 (1 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 107 | H | $CH_3$ | H | $C_6H_5$ | $C_2H_5O$ | S | O | (60 MHz) 1,38 (3 H); 2,18 (3 H); 3,62-4,5 (4 H); 5,9 (1 H); 7,4-8,1 (5 H) |
| 108 | H | $CH_3$ | H | $OCH_3$ | $NH(i\text{-}C_3H_7)$ | S | O | (60 MHz) 1,2 (6 H); 2,3 (3 H); 3,0 (1 H); 3,75 (3 H); 4,07 (2 H); 6,2 (1 H) |
| 109 | H | $CH_3$ | H | $OCH_3$ | $N(CH_3)_2$ | S | O | (60 MHz) 2,13 (3 H); 2,64 (6 H); 3,61 (3 H); 3,94 (2 H); 6,02 (1 H) |
| 110 | H | $CH_3$ | H | $OC_2H_5$ | $CH_3\text{-}C{\equiv}C\text{-}CH_2S$ | S | O | (60 MHz) 1,34 (3 H); 1,76 (3 H); 2,22 (3 H); 3,53 (2 H); 3,9-4,4 (4 H); 6,07 (1 H) |

| Nr. | $R^1$ | $R^2$ | $R^3$ | $R^4$ | $R^5$ | X | Y | NMR-Daten (MHz) δ-Werte |
|---|---|---|---|---|---|---|---|---|
| 111 | H | $C_2H_5$ | H | $OC_2H_5$ | $NH(i\text{-}C_3H_7)$ | S | O | (60 MHz) 1,2 (6 H); 1,25 (3 H); 1,92 (1 H); 2,67 (2 H); 3,68 (3 H); 4,02 (2 H); 6,11 (1 H) |

Die 1,2-Oxazolylalkylphosphate der Formel I zeigen eine gute fungizide Wirksamkeit. Sie schädigen Kulturpflanzen in den zur Bekämpfung von Pilzen und Bakterien notwendigen Konzentrationen nicht. Aus diesen Gründen sind sie als Pflanzenschutzmittel zur Bekämpfung von Pilzen geeignet. Sie können insbesondere zur Bekämpfung von Pyricularia oryzae an Reis Verwendung finden.

Die zur Bekämpfung phytopathogener Pilze erforderlichen Aufwandmengen liegen zwischen 0,05 und 5 kg Wirkstoff/ha Kulturfläche. Viele der Verbindungen sind systemisch und/oder kurativ wirksam. Sie können daher im Wasserreis in Form von Schwimmgranulaten eingesetzt werden.

Außerdem können die Wirkstoffe in die üblichen Formulierungen, wie Lösungen, Emulsionen, Suspensionen, Stäube, Pulver und Pasten übergeführt werden. Die Anwendungsformen richten sich ganz nach den Verwendungszwecken; sie sollten in jedem Fall eine feine und gleichmäßige Verteilung der wirksamen Substanzen gewährleisten. Die Formulierungen werden in bekannter Weise hergestellt, z.B. durch Verstrecken des Wirkstoffs mit Lösungsmitteln und/oder Trägerstoffen, ggf. unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen Lösungsmittel, wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe, wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen - Fettalkohol - Ether, Alkyl-

sulfonate und Arylsulfonate) und Dispergiermittel, wie Lignin, Sulfitablaugen und Methylcellulose, in Betracht.

Die fungiziden Mittel enthalten 0,1 bis 95 Gew.% Wirkstoff, vorzugsweise 0,5 bis 90 Gew.%.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile des Wirkstoffs Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung des Wirkstoffs Nr. 20 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamin, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in 100 000 Gew.-Teilen Wasser erhält man eine wäßrige Dispersion, die 0,02 Gew.% des Wirkstoffs enthält.

III. 20 Gew.-Teile des Wirkstoffs Nr. 34 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol

Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.

IV. 20 Gew.-Teile des Wirkstoffs Nr. 26 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.

V. 20 Gew.-Teile des Wirkstoffs Nr. 59 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfitablauge und 60 Gew.-Teilen pulverförmigem Kieselsäuregel gut vermischt und in einer Hammermühle vermahlen. Durch feines Verteilen der Mischung in 20.000 Gew.-Teilen Wasser erhält man eine Spritzbrühe, die 0,1 Gew.% des Wirkstoffs enthält.

VI. 3 Gew.-Teile des Wirkstoffs Nr. 82 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile des Wirkstoffs Nr. 63 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufarbeitung des Wirkstoffs mit guter Haftfähigkeit.

VIII. 40 Gew.-Teile des Wirkstoffs Nr. 45 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnungen mit 100 000 Gew.-Teilen Wasser erhält man eine wäßrige Dispersion, die 0,04 Gew.% Wirkstoff enthält.

IX. 20 Teile des Wirkstoffs Nr. 46 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die Wirkstoffe können auch mit anderen bekannten fungiziden Wirkstoffen gemischt werden. In vielen Fällen erhält man dabei auch eine Vergrößerung des fungiziden Wirkungsspektrums; bei einer Anzahl dieser Fungizidmischungen treten auch synergistische Effekte auf, d.h. die fungizide Wirksamkeit des Kombinationsproduktes ist größer als die der addierten Wirksamkeiten der Einzelkomponenten.

Fungizide, die mit den erfindungsgemäßen Verbindungen kombiniert werden können, sind beispielsweise:

Dithiocarbamate und deren Derivate, wie
Ferridimethyldithiocarbamat,
Zinkdimethyldithiocarbamat,
Manganethylenbisdithiocarbamat,
Mangan-Zink-ethylendiamin-bis-dithiocarbamat,
Zinkethylenbisdithiocarbamat,
Tetramethylthiuramdisulfide,
Ammoniak-Komplex von Zink-(N,N-ethylen-bis-dithiocarbamat) und
N,N'-Polyäthylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und
N,N'-Polypropylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Strukturen, wie
N-Trichlormethylthio-tetrahydrophthalamid,
N-Trichlormethylthio-phthalimid,
2-Heptadecyl-2-imidazolin-acetat,
2,4-Dichlor-6-(o-chloranilino)-s-triazin,
O,O-Diäthyl-phthalimidophosphonothionat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,

Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-Chlorphenyl)-3-pyridinmethanol,
1,2-Bis-(3-ethoxycarbonyl-2-thioureido)-benzol,
1,2-Bis-(3-methoxycarbonyl-2-thioureido)-benzol

und verschiedene Fungizide, wie
Dodecylguanidinacetat,
3-(3-(3,5-Dimethyl-2-oxycyclohexyl)-2-hydroxyäthyl)-glutarimid,
Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Methyl-benzoesäure-anilid,
2-Jod-benzoesäure-anilid,
1-(3,4-Dichloranilino)-1-formylamino-2,2,2-trichloräthan,
2,6-Dimethyl-N-tridecyl-morpholin bzw. dessen Salze,
2,6-Dimethyl-N-cyclododecyl-morpholin bzw. dessen Salze.

5-Nitro-isophthalsäure-di-isopropylester,
1-(1',2',4'-Triazolyl-1')-[1-(4'-chlorphenoxy)]-3,3-dimethylbutan-2-on,
1-(1',2',4'-Triazolyl-1')-[1-(4'-chlorphenoxy)]-3,3-dimethylbutan-2-ol,
N-(n-Propyl)-N-(2,4,6-trichlorphenoxyethyl)-N'-imidazolyl-harnstoff,

N-Cyclohexyl-N-methoxy-2,5-dimethyl-furan-3-carbonsäureamid,
2,4,5-Trimethyl-furan-3-carbonsäureanilid,
5-Methyl-5-vinyl-3-(3,5-dichlorphenyl)-2,4-dioxo-1,3-oxazolidin,
5-Methoxymethyl-5-methyl-3-(3,5-dichlorphenyl)-2,4-dioxo-1,3-oxazolidin,
N-[3-(p-tert.-Butylphenyl)-2-methyl-propyl]-cis-2,6-dimethylmorpholin.

Die fungiziden Mittel können auch zusammen mit anderen Wirkstoffen, z.B. mit Herbiziden, Insektiziden und Wachstumsregulatoren, oder auch mit Düngemitteln vermischt ausgebracht werden.

Die fungizide Wirksamkeit der 1,2-Oxazolylalkylphosphate wird durch den folgenden Test gezeigt. Vergleichsmittel ist O,O-Diethyl-S-benzyl-thiol-phosphat (Wegler, Chemie der Pflanzenschutz- und Schädlingsbekämpfungsmittel, Band 2, Seite 131, Springer-Verlag, Berlin, 1970).

Wirksamkeit gegen Pyricularia oryzae an Reis

Blätter von in Töpfen gewachsenen Reiskeimlingen werden mit einer wäßrigen Konidienaufschwemmung des Pilzes Pyricularia oryzae künstlich infiziert. Nach der Infektion werden die Pflanzen 24 Stunden lang in einer Kammer bei Temperaturen von 22 bis 25°C und hoher Luftfeuchtigkeit (Wasserdampfsättigung) aufgestellt und nach Ablauf dieser Zeit mit 0,1 gewichtsprozentigen wäßrigen Wirkstoffemulsionen, die 80 % Wirkstoff und 20 % Dispergiermittel in der Trockensubstanz enthalten, tropfnaß gespritzt. Die Töpfe mit den Pflanzen werden dann in die feuchte Kammer zurückgestellt.
Nach 5 Tagen haben sich auf den unbehandelten Kontrollpflanzen die Krankheitssymptome so stark ausgebildet, daß die entstandenen Blattflecken den überwiegenden Teil der Gesamtfläche bedecken.

In diesem Test zeigen die Wirkstoffe Nr. 1, 20, 25, 26, 29, 30, 34, 37, 38, 45, 46, 49, 50, 59, 60, 63, 67, 72, 74, 77, 82, 83, 85, 93 eine bessere Wirkung als das Vergleichsmittel.

## Patentansprüche

1. Fungizides Mittel, enthaltend inerte Zusatzstoffe und ein 1,2-Oxazolylalkylphosphat der Formel

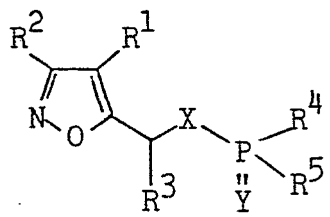

(I),

in der

$R^1$ Wasserstoff oder Halogen,

$R^2$ Wasserstoff, eine Alkyl-, eine Alkoxyalkyl- oder eine Alkylthioalkylgruppe mit bis zu 6 Kohlenstoffatomen, einen gegebenenfalls durch Alkylgruppen, Halogen oder Nitro substituierten Phenyl- oder Phenylalkylrest oder einen Alkoxycarbonylrest mit bis zu 4 Kohlenstoffatomen in der Alkoxygruppe,

$R^3$ Wasserstoff oder eine Alkylgruppe mit bis zu 3 Kohlenstoffatomen,

$R^4$ eine Alkyl- oder Alkoxygruppe mit bis zu 6 Kohlenstoffatomen oder Phenyl,

$R^5$ eine Alkoxy-, eine Alkoxyalkyl-, eine Alkylthioalkylthio-, eine Alkylthio-, Alkenylthio-, Alkinylthio-, Cycloalkoxy- oder Cycloalkylthiogruppe mit bis zu 6 Kohlenstoffatomen, Benzylthio, Amino, eine Alkylamino- oder Dialkylaminogruppe mit bis zu 4 Kohlenstoffatomen in einer Alkylgruppe und

X und Y unabhängig voneinander Sauerstoff oder Schwefel bedeuten, als Wirkstoff.

2. Verfahren zur Bekämpfung von Pilzen, dadurch gekennzeichnet, daß man eine fungizid wirksame Menge eines

1,2-Oxazolylalkylphosphats der Formel

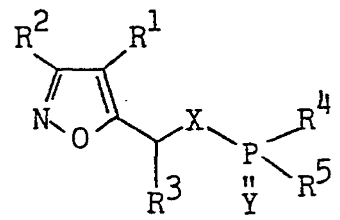 (I),

in der

$R^1$ Wasserstoff oder Halogen,

$R^2$ Wasserstoff, eine Alkyl-, eine Alkoxyalkyl- oder eine Alkylthioalkylgruppe mit bis zu 6 Kohlenstoffatomen, einen gegebenenfalls durch Alkylgruppen, Halogen oder Nitro substituierten Phenyl- oder Phenylalkylrest oder einen Alkoxycarbonylrest mit bis zu 4 Kohlenstoffatomen in der Alkoxygruppe,

$R^3$ Wasserstoff oder eine Alkylgruppe mit bis zu 3 Kohlenstoffatomen,

$R^4$ eine Alkyl- oder Alkoxygruppe mit bis zu 6 Kohlenstoffatomen oder Phenyl,

$R^5$ eine Alkoxy-, eine Alkoxyalkyl-, eine Alkylthioalkylthio-, eine Alkylthio-, Alkenylthio-, Alkinylthio-, Cycloalkoxy- oder Cycloalkylthiogruppe mit bis zu 6 Kohlenstoffatomen, Benzylthio, Amino, eine Alkylamino- oder Dialkylaminogruppe mit bis zu 4 Kohlenstoffatomen in einer Alkylgruppe und

X und Y unabhängig voneinander Sauerstoff oder Schwefel bedeuten,

auf durch Pilzbefall bedrohte Flächen, Pflanzen oder Saatgüter oder auf Pilze einwirken läßt.

3. Verfahren nach Anspruch 2, dadurch gekennzeichnet, daß die Aufwandmenge an 1,2-Oxazolylalkylphosphat der

Formel I 0,05 bis 5,0 kg/ha beträgt.

4. Verwendung von 1,2-Oxazolylalkylphosphaten der Formel I gemäß Anspruch 1 zur Bekämpfung von Pilzen.

Europäisches Patentamt

**0061037**

Nummer der Anmeldung

# EUROPÄISCHER RECHERCHENBERICHT

EP 82 10 1712

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | DE-A-2 549 962 (BASF) * Seite 2, Absatz 1; Seite 5, Zeile 30 - Seite 6, Zeile 8 * | 1,2,4 | A 01 N 57/08<br>C 07 F 9/65<br>C 07 D 261/06 |
| X | DE-A-2 549 961 (BASF) * Anspruch 1; Seite 12, Zeile 22 - Seite 14, Zeile 5 * & US - A - 4 212 861 (Cat. D) | 1,2,4 | |
| D,X | GB-A-1 261 158 (SHELL) * Seite 1, Zeilen 10-35; Beispiel 5 * | 1-4 | |
| X | DE-A-1 770 936 (HOECHST) * Beispiel 10; Anspruch 8 * | 1-4 | |
| A | CHEMICAL ABSTRACTS; Band 86, Nr. 19, 9. Mai 1977, Seite 162, Nr. 134875y, Columbus, OHio, USA & JP - A - 76 139 629 (SANKYO CO., LTD.) 02-12-1976 * Zusammenfassung * | 1-4 | |

RECHERCHIERTE SACHGEBIETE (Int. Cl. 3)

A 01 N
C 07 F
C 07 D

Der vorliegende Recherchenbericht wurde für alle Patentansprüche erstellt.

| Recherchenort | Abschlußdatum der Recherche | Prüfer |
|---|---|---|
| DEN HAAG | 15-06-1982 | FLETCHER A.S. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82